# EUROPEAN PATENT SPECIFICATION

(11) **EP 3 159 011 B1**
(45) Date of publication and mention of the grant of the patent: **18.03.2020**
(21) Application number: 16194896.3
(22) Date of filing: 20.10.2016
(51) Int. Cl.: A61K 9/08, A61K 47/02, A61K 47/10, A61K 47/32, A61K 47/36, A61K 31/727, A61K 31/728, A61K 31/737, A61P 41/00

(54) **OPHTHALMIC COMPOSITION**
OPHTHALMISCHE ZUSAMMENSETZUNG
COMPOSITION OPHTALMIQUE

(30) Priority: 20.10.2015 IT UB20154802
(43) Date of publication of application: 26.04.2017
(73) Proprietor: Medivis S.R.L., 95127 Catania (CT) (IT)
(72) Inventor: VINCIGUERRA, Paolo, 20136 MILANO (IT); VINCIGUERRA, Riccardo, 20136 MILANO (IT); MANGIAFICO, Sebastiano, 95127 CATANIA (IT); MANGIAFICO, Sergio, 95127 CATANIA (IT)
(74) Representative: Croce, Valeria

(56) References cited:
- EP-A1- 2 057 983
- WO-A1-99/13863
- WO-A2-2005/084635
- WO-A2-2007/030623
- US-A1- 2009 152 306
- US-A1- 2013 338 240
- LIESEGANG ET AL: "Viscoelastic substances in ophthalmology", SURVEY OF OPHTHALMOLOGY, vol. 34, no. 4, 1 January 1990 (1990-01-01), pages 268-293, XP026333141, ISSN: 0039-6257, DOI: 10.1016/0039-6257(90)90027-S [retrieved on 1990-01-01]

## Description

### Description

The invention is defined by the claims. Any subject-matter falling outside the scope of the claims is provided for information purposes only.

The present application relates to an ophthalmic composition which is an aqueous composition having pH between 7 and 7.4, osmolarity between 280 and 300 mOsm/1, and which comprises one or more glycosaminoglycans (GAGs) in amounts ranging between 0.1 and 5% w/w, polyvinyl alcohol (PVA) in a concentration between 0.1 and 0.5% w/w, glycerol in amounts ranging between 0.5 and 1% w/w, sodium chloride between 0.01 and 1% w/w, disodium phosphate between 0.1 and 1% w/ w, wherein said composition has a contact angle measured over a Kapton surface in the range of 50° to 80°, wherein said contact angle is measured as a static contact angle by means of the sessile drop method with the instrument Contact Angle System OCAE15, wherein the drop has a volume of µl and the deposition rate is 1 µl/s, wherein the measurement time after the drop deposition is 50 seconds.

In a further aspect, the present invention relates to the above composition for use in epithelial protection in femto-assisted eye surgery and to the above composition for use in femto-assisted cataract eye surgery.

### Background art

Modern eye surgery involves an increasingly wide use of femtosecond laser. Femtosecond lasers allow great improvements, though entailing major technical difficulties.

In particular, the docking step, i.e. the step where the contact between the eye, in the frontmost part thereof, the cornea and the laser optics occurs, is of the utmost importance for the success of the whole procedure. This contact must be stable during the surgery and, to this end, suction is applied to the limbus in order to form a unit between laser and eye. In order to achieve the best contact it is also essential to properly place the patient, considering that the iris plane should be as parallel as possible to the laser plane, in order to prevent the bulb from tilting, which may result in difficult applanation and suction, with possible loss of suction and need to repeat it and cause subconjunctival hemorrhage, miosis and inflammation.

The major difficulty encountered in this step is the lack of uniform contact between the cornea and the laser optics, caused by the difference between the corneal curvature and the laser optics curvature. This causes an optical inhomogeneity caused by the formation of air bubbles which are formed where the curvatures are not matching. The air bubbles do not allow the laser radiation to pass in a predictable and efficient manner, whereby the surgery cannot start. Therefore, the laser-cornea interface represents a fundamental aspect of the efficiency of treatment, since it must be free of air bubbles, should not damage the epithelial surface, should allow the passage of the infrared laser beam, and should adapt to the shape difference between the cornea and the laser optical surface.

Solutions proposed in the prior art to obtain a laser-cornea interface with the desired features include repeated attempts by the surgeon until a contact surface without bubbles is obtained, each attempt being associated with possible drawbacks.

US 2013/338240 A1 discloses a viscoelastic fluid for protecting the cornea from damages to the epithelium during eye surgery, such as cataract surgery, said fluid comprising 1.54% w/ w hyaluronic acid, 0.82% w/w sodium chloride and 0.07% w/w disodium phosphate. The pH of the composition is of 6.8-7.6, and the osmolarity is of 280-330 mOsm/l.

The use of saline has also been proposed as an interface between cornea and laser, which however entails some drawbacks, since by laminating into thin layers, said saline easily cause air bubbles, where the shape discrepancy between the contact surfaces is often greater than the thickness of the liquid foil formed. Moreover, said solution does not have an adequate corneal permanence time, therefore the surgeon must repeatedly irrigate the epithelium, thus damaging it and reducing the physiological transparency thereof. Finally, said saline dries quickly, thus allowing that the docking is carried out directly on the corneal surface, thereby creating inflammation, subconjunctival hemorrhage, and intra-operative miosis. The post-surgery visual recovery, even using the saline described in the prior art, remains difficult.

Therefore, a need is strongly felt for a composition which allows to carry out ophthalmic surgery with femtosecond laser in an advantageous and safe manner, allowing to obtain a laser-cornea interface which facilitates the adaptation of the shape difference between the cornea and the laser optical surface, without the formation of air bubbles and without damage to the epithelial surface.

### Description of the invention

The present invention has surprisingly shown that an ophthalmic composition containing glycosaminoglycans is able to achieve an unexpected improvement of cataract surgery with femtosecond laser, which improvement is observed both under the technical profile (handling, duration of surgery) and under the therapeutic profile (corneal protection, postoperative visual recovery).

The composition of the present invention is a viscous composition, with a non-Newtonian behavior, and allows to create a contact surface which, upon approaching the cornea by the laser optics, forms the first contact by pushing out the air bubbles present in the interface, thus obtaining a perfect laser-cornea adherence and the best condition for the laser radiation to pass.

### Detailed description of the invention

The composition of the present invention is an ophthalmic composition which is an aqueous composition having pH between 7 and 7.4, osmolarity between 280 and 300 mOsm/1, and which comprises one or more glycosaminoglycans (GAGs) in amounts ranging between 0.1 and 5% w/w, polyvinyl alcohol (PVA) in a concentration between 0.1 and 0.5% w/w, glycerol in amounts ranging between 0.5 and 1% w/w, sodium chloride between 0.01 and 1% w/w, disodium phosphate between 0.1 and 1% w/w, wherein said composition has a contact angle measured over a Kapton surface in the range of 50° to 80°, wherein said contact angle is measured as a static contact angle by means of the sessile drop method with the instrument Contact Angle System OCAE15, wherein the drop has a volume of 3 µl and the deposition rate is 1 µl/s, wherein the measurement time after the drop deposition is 50 seconds.

Said one or more glycosaminoglycans are preferably selected from the group comprising: chondroitin sulfate, dermatan sulfate, keratan sulfate, heparin, heparan sulfate, hyaluronic acid.

Preferably, said composition has osmolarity between 290 and 295 mOsm/l and a contact angle measured over a Kapton surface in the range of 55° and 70°. Even more preferably, said contact angle is in the range between about 58° and about 65°.

In a preferred embodiment, said composition comprises: one or more GAGs in amounts between 0.2% and 3% w/w, glycerol in amounts ranging between 0.7 and 0.9% w/w, sodium chloride in concentrations between 0.1 and 0.3% w/w, disodium phosphate in concentrations between 0.2 and 0.5% w/w; even more preferably, said one or more GAGs are present in a concentration of about 0.25% w/w, said glycerol of about 0.8% w/w, sodium chloride of 0.18% w/w, disodium phosphate of 0.3% w/w.

In a further preferred embodiment, said GAG is hyaluronic acid (HA).

In an even more preferred embodiment, said aqueous composition having pH between 7 and 7.4, osmolarity between 280 and 300 mOsm/l, and which comprises one or more glycosaminoglycans (GAGs), wherein said composition has a contact angle measured over a Kapton surface by means of the sessile drop method with the instrument Contact Angle System OCAE15 in the range of about 50° and 80°, also comprises polyvinyl alcohol in a concentration between 0.15 and 0.5% w/w, even more preferably between 0.2 and 0.4% w/w.

A preferred composition according to the present invention is a composition consisting of:

| | % w/w |
|---|---|
| **Hyaluronic acid (HA)** | 0.25 |
| **Polyvinyl alcohol (PVA)** | 0.25 |
| **Glycerol** | 0.8 |
| **NaCl** | 0.18 |
| **Disodium phosphate** | 0.3 |
| **Purified water** | as needed 100 |
| **pH** | 7.23 |
| **Osmolarity (mOsm/l)** | 292 |

A further aspect of the present invention is an aqueous composition as described above for use in epithelial protection in femto-assisted eye surgery.

Preferably, said surgery is a surgery performed in the presence of cataracts.

An eye treatment method is also described, which comprises:
a) Instilling into the eye of a subject in need thereof the above-mentioned aqueous composition;
b) Performing the docking;
c) Proceeding with the laser surgery.

The advantages resulting from the use of a composition according to the present invention are observable at the level of clinical, physical and external parameters. A considerable reduction in the epithelial damage consequent to the surgery as well as a slow epithelial evaporation during the surgery itself are observed. The formation of air bubbles between the cornea and the laser optics is significantly decreased, with a consequent reduction in the surgery duration and increased patient's compliance. The surprising synergy shown by the combination of one or more glycosaminoglycans with polyvinyl alcohol results in the absolute absence of epithelial damage, with excellent corneal transparency and a consequent rapid post-surgery recovery of the visual acuity. The epithelial evaporation during surgery is not observable. The air bubbles between the cornea and the laser optics are entirely absent, with a complete compensation for curvature irregularities where the foil formed is thick and constant. The surgical procedure is facilitated, thereby minimizing the execution times and optimizing the patient's compliance.

A further advantage is the absence of low molecular weight surfactants in the composition of the present invention, which are toxic to the ocular surface, in particular to the corneal epithelium.

### Examples

### Example 1. Preparation of the compositions wherein composition 1 is according to the invention and compositions 2 and 3 are not according to the invention.

An osmoprotectant was weighed in a dedicated container and was diluted in water.

To the solution thus obtained, the polymers according to the formulation examples shown in table 1 were added at room temperature followed by other excipients. The whole was kept under stirring at 50 rpm to complete dissolution.

To the polymer solution, disodium phosphate was then added for pH adjustment. The solution was then kept under stirring for at least 35 minutes.

**Table 1. Composition of compositions 1-3**

| **COMPOSITION** | **1** | **2** | **3** |
|---|---|---|---|
| | % w/w | % w/w | % w/w |
| **Hyaluronic acid (HA)** | 0.25 | 0.25 | - |
| **Polyvinyl alcohol (PVA)** | 0.25 | - | 0.25 |
| **Glycerol** | 0.8 | 0.8 | 0.8 |
| **NaCl** | 0.18 | 0.18 | 0.18 |
| **Disodium phosphate** | 0.3 | 0.3 | 0.3 |
| **Purified water** | as needed to 100 | as needed to 100 | as needed to 100 |
| **pH** | 7.23 | 7.25 | 7.21 |
| **Osmolarity (mOsm/l)** | 292 | 294 | 290 |

### Example 2. Contact angle measurement over a Kapton surface.

The reference surfaces considered for the purposes of the present invention were: plexiglass (polymethylmethacrylate) and Kapton (polyimide) both with the same surface tension, but the Kapton surface provided with a greater number of heteroatoms and therefore a greater capacity to establish hydrogen bonds.

The surface tension of the cornea in the presence of mucus is about 40 mN/m. This result is in agreement with the surface tension obtained by the Zisman's method in previous studies on the wettability and on the corneal surface tension (Zisman WA, 1964 "Relation of equilibrium contact angle to liquid and solid constitution". Advances in Chemistry. No 43, Washington DC: American Chemical Society, pp. 1 - 51; Sharma A, 1993 "Energetics of corneal epithelial cell-ocular mucus-tear film interactions: some surface-chemical pathways of corneal defense". Biophys Chem., Jul;47(1):87-99; Holly FJ, Lemp MA, 1971 "Wettability and wetting of corneal epithelium".Exp Eye Res., Mar;11(2) :239-50).

The surface tension of plexiglass and kapton is about 38-40mN/m. Given the similarity with the surface tension of the cornea in the presence of mucus, said two surfaces were selected as reference surfaces.

The contact angle measurements on Plexiglass showed a significant advantage when using the glycosaminoglycan + polyvinyl alcohol combination. The contact angle measured on Plexiglass is 70° ± 4° for composition 2, thus in the absence of PVA, and 58° ± 3° for composition 1, thus with the glycosaminoglycan + PVA combination. The measurements were carried out by using the Contact Angle System OCAE15, with the sessile drop method, with a dispensed volume of 1 µL.

The measurements have great validity from a comparative point of view and with no doubt, the difference is indicative of an advantage on the cornea wettability. Considering that the surface tension values determined experimentally by the Zisman's method mainly take into account the non-polar component of the surface tension and exclude the polar component which also affects the wettability, a second hydrophobic surface (the Kapton) was also evaluated.

Table 2 shows the mean results obtained for each of the compositions 1 to 3 tested, following five independent measurements of the contact angle, which measurements were carried out two seconds after the drop deposition on the Kapton surface. The measurements were carried out by using the Contact Angle System OCAE15, with the sessile drop method, with a dispensed volume of 1 µL.

**Table 2. Contact angle over Kapton**

| **Saline** | **Composition 1** | **Composition 2** | **Composition 3** |
|---|---|---|---|
| **Kapton** | **Kapton** | **Kapton** | **Kapton** |
| 85° ± 4 | 60° ± 3 | 80° ± 4 | 50° ± 3 |

As apparent from table 2, the average contact angle of Composition 1 (containing HA and PVA) has a lower value than that of Composition 2 (containing HA) but higher than that of Composition 3 (containing PVA alone). The saline gives the greatest, i.e. unfavorable, contact angle.

### Example 3. Clinical evaluation of the effect of Compositions 1-3 in patients undergoing femto-assisted cataract eye surgery.

40 patients, divided into four groups, were involved: A, B, C and D. Group A was instilled with a balanced saline prior to the docking between cornea and laser. The saline used consists of an aqueous solution containing 0.18% w/w NaCl. Groups B, C and D were instead instilled with Compositions 1, 2 and 3, respectively, described in example 1. The parameters measured were of clinical type: epithelial damage, subconjunctival hemorrhage, post-surgery visual acuity, epithelial evaporation, corneal transparency; physical type: thickness of the liquid foil, formation of air bubbles, compensation for curvature irregularities, intra-operative visibility; and external type: patient's compliance, ease of execution for the surgeon, procedure duration. The results of the evaluation carried out are summarized in Table 3.

**Table 3. Results of the clinical evaluation in patients undergoing femto-assisted eye cataract surgery with the use of: Saline (Group A), Composition 1 (Group B), Composition 2 (Group C), Composition 3 (Group D)**

| | **Saline (A)** | **Composition 1 (B)** | **Composition 2 (C)** | **Composition 3 (D)** |
|---|---|---|---|---|
| **Clinical parameters** | | | | |
| Epithelial damage | Damaged | Not damaged | Little damaged | Little damaged |
| Subconjunctival hemorrhage | Frequent and extended | Small and circumscribed | Frequent and extended | Frequent and extended |
| Epithelial evaporation | Fast | Not observable | Observable | Observable |
| Corneal transparency | Compromised | Excellent | Compromised | Compromised |
| Post-surgery visual acuity | Late recovery | Fast | Late recovery | Late recovery |

| **Physical parameters** | | | | |
|---|---|---|---|---|
| Liquid foil thickness | Thin and irregular | Thick and constant | Thin and irregular | Thin and irregular |
| Formation of air bubbles | Frequent | Absent | Present | Present |
| Curvature irregularity compensation | Almost absent | Always | Almost absent | Almost absent |
| Intra-operative visibility | Compromised | Excellent | Compromised | Compromised |

| **External parameters** | | | | |
|---|---|---|---|---|
| Patient's collaboration | Very little | Excellent | Little | Little |
| Ease of execution for the surgeon | Difficult | Easy | Difficult | Difficult |
| Surgery duration | Long | Medium | Medium | Long |

Surprisingly, composition 1, which comprises a combination of Hyaluronic acid and Polyvinyl alcohol, appears to be the most effective, not only with respect to the prior art saline, but also to composition 2 containing no PVA and 3, which does not contain hyaluronic acid, thus showing a clear synergistic effect of combining hyaluronic acid and polyvinyl alcohol.

In particular, in patients belonging to Group A, for whom the surgeon had used the Composition 1 of the present invention, during the femto-assisted surgery, it is noted that said formulation is able to: protect the corneal epithelium, reduce subendothelial hemorrhage which when present are circumscribed, ensure an excellent corneal transparency, and allow fast post-surgery recovery of the visual acuity. Moreover, composition 1 is found to have the best physical characteristics during the surgery, in that: the liquid foil on the cornea is thicker and remains constant, the formation of air bubbles is absent, is able to compensate for corneal curvature irregularities, and provides excellent intra-operative visibility. Composition 1 is surprisingly able to clearly influence those parameters which are unchanged in the presence of only one of the two components, Hyaluronic acid or Polyvinyl alcohol. In fact, it is only the synergy between hyaluronic acid and polyvinyl alcohol which is able to contribute to a significant decrease compared to the observed onset of subconjunctival hemorrhage, to the impairment of the corneal transparency, and which allows fast post-surgery recovery of the visual acuity. And again, it is only the synergy which allows the formation of a thick and constant liquid foil, again capable of compensating for curvature irregularities, and which allows a good intra-operative visibility, with a consequent ease of execution for the surgeon.

## Claims

1. An ophthalmic composition which is an aqueous composition having pH between 7 and 7.4, osmolarity between 280 and 300 mOsm/l, and which comprises one or more glycosaminoglycans (GAGs) in amounts ranging between 0.1 and 5% w/w, polyvinyl alcohol (PVA) in a concentration between 0.1 and 0.5% w/w, glycerol in amounts ranging between 0.5 and 1% w/w, sodium chloride between 0.01 and 1% w/w, disodium phosphate between 0.1 and 1% w/w, wherein said composition has a contact angle measured over a Kapton surface in the range of 50° to 80°, wherein said contact angle is measured as a static contact angle by means of the sessile drop method with the instrument Contact Angle System OCAE15, wherein the drop has a volume of 3 µl and the deposition rate is 1 µl/s, wherein the measurement time after the drop deposition is 50 seconds.

2. A composition according to claim 1, wherein said osmolarity is in the range between 290 and 295 mOsm/l and said contact angle is in the range between 55° and 70°, preferably between 58° and 65°.

3. A composition according to one of claims 1 or 2, comprising: one or more GAGs between 0.2% and 3% w/w, glycerol between 0.7 and 0.9% w/w, sodium chloride between 0.1 and 0.30 w/w, disodium phosphate in concentrations between 0.2 and 0.5% w/w; preferably, said one or more GAGs are present in a concentration of 0.25% w/w, said glycerol of 0.8% w/w, sodium chloride of 0.18% w/w, disodium phosphate of 0.3% w/w.

4. A composition according to any one of claims 1 to 3, wherein said GAG is hyaluronic acid.

5. A composition according to any one of claims 1 to 4, wherein said PVA is in a concentration between 0.15 and 0.5% w/w, even more preferably between 0.2 and 0.4% w/ w.

6. A composition according to any one of claims 1 to 5, consisting of: 0.25% w/w hyaluronic acid, 0.25% w/w polyvinyl alcohol, 0.8% w/w glycerol, 0.18% w/w sodium chloride, 0.3% w/w disodium phosphate, and purified water as needed to 100% w/w, which has pH of 7.23 and osmolarity of 292 mOsm/l.

7. A composition according to any one of claims 1 to 6, for use in epithelial protection in femto-assisted eye surgery.

8. A composition according to any one of claims 1 to 6, for use in femto-assisted cataract eye surgery.

## Patentansprüche

1. Ophthalmische Zusammensetzung, die eine wässrige Zusammensetzung ist mit einem pH-Wert zwischen 7 und 7,4, einer Osmolarität zwischen 280 und 300 mOsm/l und die ein oder mehrere Glycosaminoglycane (GAGs) in Mengen von zwischen 0,1 und 5 Gew.-%, Polyvinylalkohol (PVA) in einer Konzentration zwischen 0,1 und 0,5 Gew.-%, Glycerin in Mengen von zwischen 0,5 und 1 Gew.-%, Natriumchlorid zwischen 0,01 und 1 Gew.-%, Dinatriumphosphat zwischen 0,1 und 1 Gew.-% umfasst,
wobei die Zusammensetzung einen Kontaktwinkel aufweist, der über einer Kapton-Oberfläche im Bereich von 50° bis 80° gemessen wird, wobei der Kontaktwinkel als ein statischer Kontaktwinkel mittels des Sessile-Drop-Verfahrens mit dem Instrument Kontaktwinkel-System OCAE15 gemessen wird, wobei der Tropfen ein Volumen von 3 µl aufweist und die Abscheiderate 1 µl/s beträgt, wobei die Messzeit nach der Tropfenabscheidung 50 Sekunden beträgt.

2. Zusammensetzung nach Anspruch 1, wobei die Osmolarität im Bereich zwischen 290 und 295 mOsm/l liegt und der Kontaktwinkel im Bereich zwischen 55° und 70°, bevorzugt zwischen 58° und 65° liegt.

3. Zusammensetzung nach einem der Ansprüche 1 oder 2, umfassend: ein oder mehrere GAGs zwischen 0,2 Gew.-% und 3 Gew.-%, Glycerin zwischen 0,7 und 0,9 Gew.-%, Natriumchlorid zwischen 0,1 und 0,3 Gew.-%. Dinatriumphosphat in Konzentrationen zwischen 0,2 und 0,5 Gew.-%; bevorzugt wobei das eine oder die mehreren GAGs in einer Konzentration von 0,25 Gew.-%, das Glycerin von 0,8 Gew.-%, Natriumchlorid von 0,18 Gew.-%, Dinatriumphosphat von 0,3 Gew.-% vorliegen.

4. Zusammensetzung nach einem der Ansprüche 1 bis 3, wobei das GAG Hyaluronsäure ist.

5. Zusammensetzung nach einem der Ansprüche 1 bis 4, wobei das PVA in einer Konzentration zwischen 0,15 und 0,5 Gew.-%, noch stärker bevorzugt zwischen 0,2 und 0,4 Gew.-% vorliegt.

6. Zusammensetzung nach einem der Ansprüche 1 bis 5, bestehend aus: 0,25 Gew.-% Hyaluronsäure, 0,25 Gew.-% Polyvinylalkohol, 0,8 Gew.-% Glycerin, 0,18 Gew.-% Natriumchlorid, 0,3 Gew.-% Dinatriumphosphat und gereinigtes Wasser nach Bedarf zu 100 Gew.-%, die einen pH-Wert von 7,23 und eine Osmolarität von 292 mOsm/l aufweist.

7. Zusammensetzung nach einem der Ansprüche 1 bis 6 zur Verwendung beim Epithelschutz bei der femtoassistierten Augenoperation.

8. Zusammensetzung nach einem der Ansprüche 1 bis 6 zur Verwendung bei der femtoassistierten Katarakt-Augenoperation.

## Revendications

1. Composition ophtalmique qui est une composition aqueuse ayant un pH entre 7 et 7,4, une osmolarité entre 280 et 300 mOsm/l, et qui comprend un ou plusieurs glycosaminoglycanes (GAGs) en des quantités se situant dans une plage entre 0,1 % et 5 % p/p, de l'alcool polyvinylique (PVA) en une concentration entre 0,1 et 0,5 % p/p, du glycérol en des quantités se situant dans une plage entre 0,5 et 1 % p/p, du chlorure de sodium entre 0,01 et 1 % p/p, de l'hydrogénophosphate de sodium entre 0,1 et 1 % p/p, dans laquelle ladite composition a un angle de contact mesuré sur une surface de Kapton dans la plage de 50° à 80°, dans laquelle ledit angle de contact est mesuré en tant qu'angle de contact statique au moyen du procédé de la goutte sessile avec l'instrument Contact Angle System OCAE15, dans laquelle la goutte a un volume de 3 µl et la vitesse de dépôt est de 1 µl/s, dans laquelle le temps de mesure après le dépôt de la goutte est de 50 secondes.

2. Composition selon la revendication 1, dans laquelle ladite osmolarité est dans la plage entre 290 et 295 mOsm/l et ledit angle de contact est dans la plage entre 55° et 70°, de préférence entre 58° et 65°.

3. Composition selon l'une des revendications 1 ou 2, comprenant : un ou plusieurs GAGs entre 0,2 % et 3 % p/p, du glycérol entre 0,7 et 0,9 % p/p, du chlorure de sodium entre 0,1 et 0,3 % p/p, de l'hydrogénophosphate de sodium en des concentrations entre 0,2 et 0,5 % p/p ; de préférence, lesdits un ou plusieurs GAGs sont présents en une concentration de 0,25 % p/p, ledit glycérol de 0,8 % p/p, le chlorure de sodium de 0,18 % p/p, l'hydrogénophosphate de sodium de 0,3 % p/p.

4. Composition selon l'une quelconque des revendications 1 à 3, dans laquelle ledit GAG est l'acide hyaluronique.

5. Composition selon l'une quelconque des revendications 1 à 4, dans laquelle ledit PVA est en une concentration entre 0,15 et 0,5 % p/p, de manière encore davantage préférée entre 0,2 et 0,4 % p/p.

6. Composition selon l'une quelconque des revendications 1 à 5, constituée de : 0,25 % p/p d'acide hyaluronique, 0,25 % p/p d'alcool polyvinylique, 0,8 % p/p de glycérol, 0,18 % p/p de chlorure de sodium, 0,3 % p/p d'hydrogénophosphate de sodium, et d'eau purifiée selon les besoins pour compléter à 100 % p/p, qui a un pH de 7,23 et une osmolarité de 292 mOsm/l.

7. Composition selon l'une quelconque des revendications 1 à 6, pour une utilisation dans une protection épithéliale dans une chirurgie oculaire assistée par femto.

8. Composition selon l'une quelconque des revendications 1 à 6, pour une utilisation dans une chirurgie oculaire de la cataracte assistée par femto.
